(19) **Europäisches Patentamt**
**European Patent Office**
**Office européen des brevets**

(11) **EP 3 662 841 B1**

(12) **EUROPEAN PATENT SPECIFICATION**

(45) Date of publication and mention
of the grant of the patent:
**15.03.2023 Bulletin 2023/11**

(21) Application number: **19214088.7**

(22) Date of filing: **06.12.2019**

(51) International Patent Classification (IPC):
**A61B 8/08** (2006.01)     **A61B 8/12** (2006.01)
**A61B 8/00** (2006.01)     **A61N 7/00** (2006.01)

(52) Cooperative Patent Classification (CPC):
**A61B 8/085; A61B 5/062; A61B 5/1075;**
**A61B 8/0883; A61B 8/12; A61B 8/4254;**
**A61B 8/5207; A61B 8/5223;** A61B 8/58;
A61B 2018/00351; A61B 2018/00577;
A61B 2034/2051; A61B 2034/2053;
A61B 2034/2061

(54) **MAPPING ENDOCARDIAL SUB-SURFACE CHARACTERISTICS**

ABBILDUNG VON ENDOKARDIALEN UNTEROBERFLÄCHENEIGENSCHAFTEN

CARTOGRAPHIE DE CARACTÉRISTIQUES DE SOUS-SURFACE ENDOCARDIAQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **07.12.2018 US 201862776841 P**
**04.11.2019 US 201916673025**

(43) Date of publication of application:
**10.06.2020 Bulletin 2020/24**

(73) Proprietor: **Biosense Webster (Israel) Ltd.**
**Yokneam, 2066717 (IL)**

(72) Inventors:
• **AGARWAL, Amit**
**Irvine, CA 92618 (US)**
• **URMAN, Roy**
**Irvine, CA 92618 (US)**

(74) Representative: **Carpmaels & Ransford LLP**
**One Southampton Row**
**London WC1B 5HA (GB)**

(56) References cited:
**EP-A2- 1 720 038**     **WO-A1-2014/064577**
**WO-A2-2012/122517**     **US-A1- 2003 013 958**
**US-A1- 2011 028 848**

• **CULSHAW B ET AL: "The Detection of Ultrasound**
**Using Fiber-Optic Sensors", IEEE SENSORS**
**JOURNAL, IEEE SERVICE CENTER, NEW YORK,**
**NY, US, vol. 8, no. 7, 1 July 2008 (2008-07-01),**
**pages 1360-1367, XP011231348, ISSN:**
**1530-437X, DOI: 10.1109/JSEN.2008.927240**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

This invention relates generally to measurements of biological tissue, and specifically to measuring a thickness of the tissue.

**BACKGROUND OF THE INVENTION**

During medical procedures performed on tissue of a patient, such as acquisition of a biopsy or ablation of a portion of the myocardium, it may be useful to know characteristics, including thickness, of the tissue. In a number of cases the characteristics may be deduced from a pre-acquired image of the tissue, such as from an MRI (magnetic resonance imaging) or a CT (computerized tomography) image. In the case of ablation, this data may not be available to the professional performing the ablation. Even if it is available, it may not give a characteristic such as the thickness to sufficient accuracy, or the thickness may have changed since acquisition of the image.

EP 1720038 A2 relates to a system and method for imaging a target in a patient's body that uses a pre-acquired image of the target and a catheter having a position sensor and an ultrasonic imaging sensor. The catheter is placed in the patient's body and positional information of a portion of the catheter in the patient's body is determined using the position sensor. The catheter is used to generate an ultrasonic image of the target using the ultrasonic imaging sensor.

WO 2014/064577 A1 relates to a spatial configuration determination apparatus for determining a spatial configuration in the surrounding of an ultrasound device, in particular, for determining the orientation of the ultrasound device and of an object in the surrounding of the ultrasound device with respect to each other.

US 2003/013958 A1 relates to apparatus for mapping a surface of a cavity within a body of a subject that includes an elongate probe, having a longitudinal axis and including a distal portion adapted for insertion into the cavity. A primary acoustic transducer on the distal portion of the probe is adapted to emit acoustic waves while the probe is in the cavity.

US 2011/028848 A1 relates to a device for measuring a spatial location of a tissue surface, such as the interface between different types of tissues or between tissue and body fluids, that includes an elongate catheter body having a distal end portion, a plurality of localization elements carried by the distal end portion, and at least one pulse-echo acoustic element carried by the distal end portion.

Culshaw B et al "The detection of ultrasound using Fiber-Optic Sensors" (IEEE sensors journal vol. 8, no. 7, 1 July 2008, pages 1360-1367) relates to a summary of the interaction mechanisms between ultrasound and fiber sensors and confirms their functional flexibility. The results demonstrate the practical use of these sensors to detect and locate damage in a sample.

WO 2012/122517 A2 relates to determining the dipole densities on heart walls. In particular, a triangularization of the heart wall is performed in which the dipole density of each of multiple regions correlate to the potential measured at various located within the associated chamber of the heart.

**SUMMARY OF THE INVENTION**

An embodiment of the present invention provides apparatus, consisting of:

a probe having a distal end configured to be brought into proximity with tissue of an internal organ of a human patient;
a position sensor, attached to the distal end and configured to generate position signals indicative of a location and an orientation of the distal end;
an ultrasonic transducer configured to transmit an ultrasound pulse in a direction of the orientation of the distal end and to receive reflections of the ultrasound pulse from the tissue;
a plurality of location sensors, attached to the distal end of the probe and configured to contact a surface of the tissue at respective locations in a vicinity of the distal end and to output respective location signals indicative of the respective locations; and
a processor, configured to
identify first and second reflections of the ultrasound pulse that were received respectively from front and rear surfaces of the tissue;
process the location signals to find an orientation angle of the front surface of the tissue in the vicinity of the distal end; and
estimate a thickness of the tissue along an axis normal to the front surface based on a time elapsed between receipt of the first and second reflections and an inclination of the direction of the orientation of the distal end relative to the orientation angle of the front surface.

In a disclosed embodiment the apparatus includes a fiber optic attached to the distal end, the fiber optic having a fiber optic proximal end, coupled to the ultrasonic transducer, and a fiber optic distal end, located at a distal termination of the distal end, configured to transmit the ultrasound pulse and receive the reflections of the ultrasound pulse. The fiber optic may have one or more optical gratings configured so that diffracted light from the gratings is indicative of the location and the orientation of the distal end, so that the one or more optical gratings act as a further position sensor.

In an alternative disclosed embodiment the position sensor includes at least one coil configured to generate the position signals in response to a magnetic field traversing the sensor. Alternatively or additionally, the position sensor includes one or more optical gratings formed in a fiber optic located in the distal end, and wherein diffracted light from the gratings is indicative of the location and the orientation of the distal end.

In a further disclosed embodiment there are at least three flexible branches splaying from a termination of the probe distal end, and the location sensors consists of electrodes attached to the flexible branches. Typically, at least one electrode is attached to each flexible branch.

In a yet further disclosed embodiment the position sensor includes at least one coil configured to generate the position signals in response to a magnetic field traversing the at least one coil.

In an alternative embodiment there are at least three flexible branches splaying from a termination of the probe distal end, and the location sensors are coils attached to the flexible branches. Typically at least one coil is attached to each flexible branch.

In a further alternative embodiment the processor is configured to calculate a position of an intersection of the front surface of the tissue with an axis of the probe based on a time of flight of the first reflection. The processor may be configured to formulate an equation for the front surface based on the position of the intersection and the respective locations. The processor may also be configured to calculate the orientation angle of the front surface at the intersection using the equation.

In a yet further alternative embodiment the processor is configured to estimate the thickness of the tissue as a projection of an apparent tissue thickness onto the axis, and the processor calculates the apparent tissue thickness in response to the time elapsed.

The internal organ is typically the heart of the human patient.

There is further provided, according to an embodiment not forming part of the present invention, a method, including:

bringing a distal end of a probe into proximity with tissue of an internal organ of a human patient;

attaching a position sensor to the distal end and generating with the sensor position signals indicative of a location and an orientation of the distal end;

transmitting, from an ultrasound transducer, an ultrasound pulse in a direction of the orientation of the distal end and receiving reflections of the ultrasound pulse from the tissue;

attaching a plurality of location sensors to the distal end of the probe and configuring the sensors to contact a surface of the tissue at respective locations in a vicinity of the distal end and to output respective location signals indicative of the respective locations;

identifying first and second reflections of the ultrasound pulse that were received respectively from front and rear surfaces of the tissue;

processing the location signals to find an orientation angle of the front surface of the tissue in the vicinity of the distal end; and

estimating a thickness of the tissue along an axis normal to the front surface based on a time elapsed between receipt of the first and second reflections and an inclination of the direction of the orientation of the distal end relative to the orientation angle of the front surface.

## BRIEF DESCRIPTION OF THE DRAWINGS

The present disclosure will be more fully understood from the following detailed description of the embodiments thereof, taken together with the drawings, in which:

Fig. 1 is a schematic illustration of an invasive medical procedure using apparatus, according to an embodiment of the present invention;

Fig. 2 is a schematic illustration of a distal end of a probe used in the apparatus, according to an embodiment of the present invention;

Fig. 3 is a schematic illustration of an alternate distal end of the probe, according to an embodiment of the present invention; and

Fig. 4 is a flowchart of steps performed for operation of the apparatus.

**DETAILED DESCRIPTION OF EMBODIMENTS**

Overview

Typically, during a procedure investigating characteristics of tissue, embodiments of the present invention provide an independent measure of a thickness of the tissue. A probe has a position sensor attached to a distal end of the probe, and a processor uses signals from the sensor to determine a location and an orientation of the probe distal end. The distal end is configured to be able to be brought into proximity with tissue of an internal organ of a human patient.

The probe has a plurality of location sensors, typically comprising electrodes, attached to its distal end, and the sensors are able to contact a surface of the tissue at respective locations in a vicinity of the distal end. The sensors output respective location signals indicative of the respective locations.

An ultrasonic transducer is configured to transmit an ultrasound pulse in a direction of the orientation of the distal end and is also able to receive reflections of the ultrasound pulse from the tissue.

The processor is configured to identify first and second reflections of the ultrasound pulse that were received respectively from front and rear surfaces of the tissue. The processor processes the location signals to find an orientation angle of the front surface of the tissue in the vicinity of the distal end. The processor then estimates a thickness of the tissue along an axis normal to the front surface based on a time elapsed between receipt of the first and second reflections and an inclination of the direction of the orientation of the distal end relative to the orientation angle of the front surface.

Detailed Description

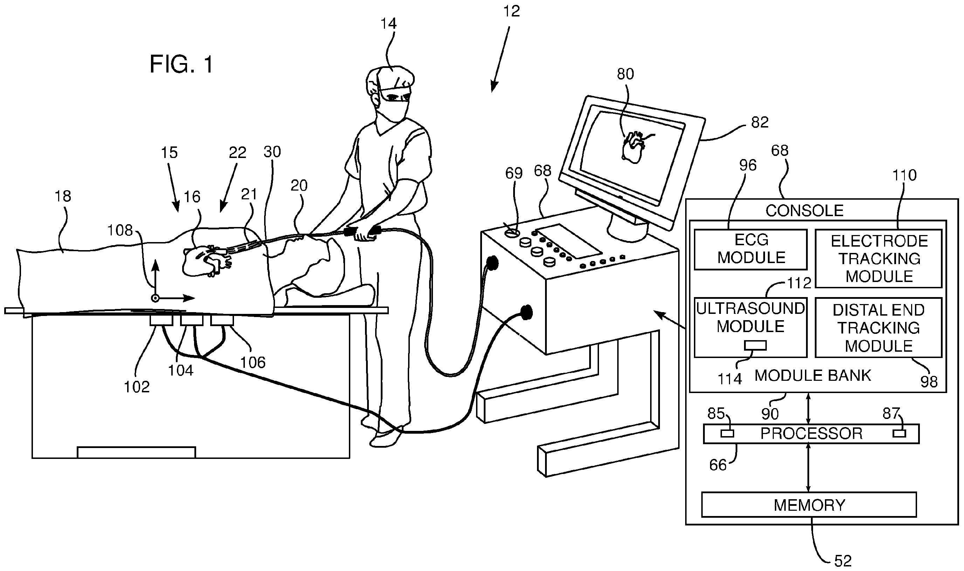

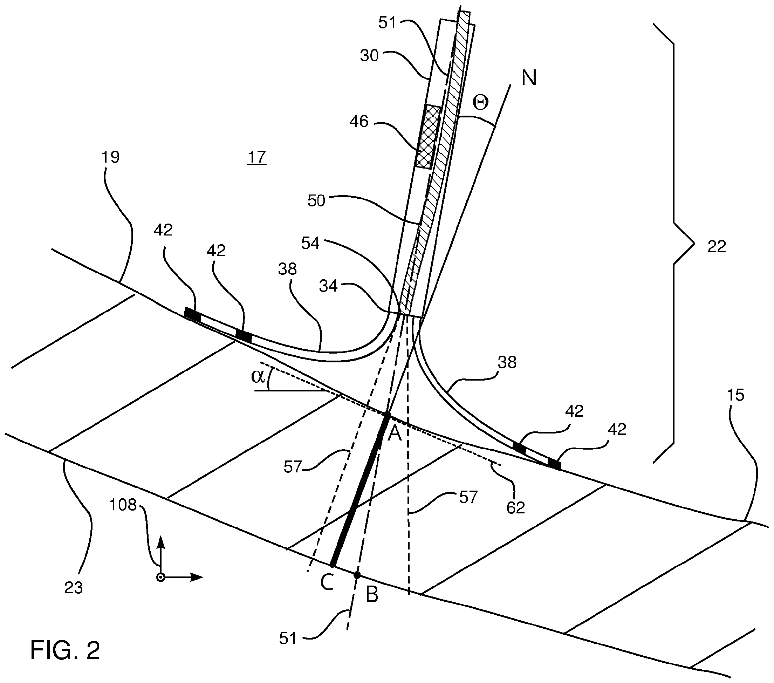

Fig. 1 is a schematic illustration of an invasive medical procedure using apparatus 12, and Fig. 2 is a schematic illustration of a distal end 22 of a probe 20 used in the apparatus, according to an embodiment of the present invention. Probe 20 acts as a catheter, and is also referred to herein as catheter 20. The procedure is performed by a medical professional 14, and in the description hereinbelow the procedure is assumed to comprise characterization of a portion of tissue 15 of an internal organ of a human patient 18, the internal organ herein being assumed, by way of example, to comprise a myocardium 16 of the heart of a human patient 18. The characterization typically includes determination of the thickness of the portion of tissue being investigated, i.e., determination of a distance between a front or proximal surface 19 of the tissue, and a rear or distal surface 23 of the tissue.

In order to perform the procedure, professional 14 inserts probe 20 into a sheath 21 that has been pre-positioned in a lumen of the patient. Sheath 21 is positioned so that distal end 22 of the probe enters the heart of the patient, and is in a region 17 surrounded by tissue 15. Region 17 contains blood being pumped by the heart.

Probe 20 comprises a single tubular element 30, which terminates at distal end 22 of the probe at a tubular termination 34. At distal end 22, three or more flexible branches 38 splay from termination 34 (for simplicity, Fig. 2 only illustrates two such branches) . In one embodiment there are five branches 38, as in the Pentaray® catheter produced by Biosense Webster of 33 Technology Drive, Irvine, CA 92618 USA, and the Pentaray® catheter may be used in a disclosed embodiment of the present invention. However, it will be understood that embodiments of the present invention may comprise any number of flexible branches greater than two. Each branch 38 comprises at least one electrode 42, and there are typically two or more electrodes 42 on each branch. In the Pentaray® catheter referred to above, there are four electrodes in each branch. In one such Pentaray catheter the spacing between each of the four electrodes is 4 mm. In another Pentaray® catheter, the spacing between the two distal electrodes is 2 mm, between the two proximal electrodes is also 2 mm, and between the two central electrodes is 6 mm.

A position sensor 46 is fixedly located within element 30, near termination 34. As is described in more detail below, the sensor generates signals which enable the location and orientation of the sensor itself, and thus of fixed elements within the distal end, to be tracked. A fiber optic 50 is incorporated within element 30, the fiber optic having a fiber optic distal end 54 at substantially the same position as termination 34. As is also described in more detail below, fiber optic 50 is configured to transmit ultrasound pulses, so that fiber optic distal end 54 acts as both a transmission port and a receiving port for the ultrasound pulses, and is herein also referred to as transceiver port 54.

Transceiver port 54 transmits the ultrasound pulses externally to distal end 22 in a narrow cone having boundaries schematically shown by broken lines 57. The cone has an axis of symmetry corresponding to an axis of symmetry axis 51 of element 30, and for simplicity in the description herein the pulses are assumed to be transmitted along axis 51. Port 54 also receives ultrasound pulses (external to the distal end) travelling along the same axis.

In some embodiments, fiber optic 50 is incorporated into an existing "off-the-shelf" catheter. For example, fiber optic 50 may be fed through the irrigation channel of an existing Pentaray® catheter. In some embodiments fiber optic 50 can also be configured, by having one or more optical gratings incorporated into the fiber optic to act as a location and orientation sensor for distal end 22. In this case fiber optic 50 uses fiber optic shape sensing (FOSS) technology, as is known in the art. The FOSS technology analyzes light diffracted from the gratings to estimate a location and an orientation of the gratings. It will be understood that if fiber optic 50 is configured as a location and orientation sensor, it may be used as well as, or in place of, position sensor 46.

Apparatus 12 is controlled by a system processor 66, which is located in an operating console 68 of the apparatus. Console 68 comprises controls 69 which are used by professional 14 to communicate with the processor. The software for processor 66 may be downloaded to the processor in electronic form, over a network, for example and stored in a memory 52 in communication with the processor. Alternatively or additionally, the software may be provided on non-transitory tangible media, such as optical, magnetic, or electronic storage media. The track of distal end 22 is typically displayed on a three-dimensional representation 80 of the heart of patient 18 that is displayed on a screen 82.

System processor 66 comprises real-time noise reduction circuitry 85, typically configured as a field programmable gate array (FPGA), followed by an analog-to-digital (A/D)) signal conversion integrated circuit 87. The processor can pass the signal from A/D circuit 87 to another processor and/or can be programmed to perform at least one algorithm disclosed herein, the algorithm comprising steps described hereinbelow. The processor uses circuitry 85 and circuit 87, as well as features of modules which are described in more detail below, in order to perform the algorithm.

In order to operate apparatus 12, the algorithm of processor 66 communicates with a module bank 90, which has a number of modules used by the processor to operate the apparatus. Thus, bank 90 comprises an electrocardiograph (ECG) module 96 which acquires and analyzes signals from electrodes 42, and a distal end tracking module 98 which receives and analyzes signals from position sensor 46, and which uses the signal analysis to generate a location and an orientation of distal end 22.

In some embodiments sensor 46 comprises one or more coils which provide the sensor signals in response to magnetic fields traversing the coils. In these embodiments, in addition to receiving and analyzing signals from sensor 46, tracking module 98 also controls radiators 102, 104, and 106 which radiate the magnetic fields traversing sensor 46. The radiators are positioned in proximity to myocardium 16, are configured to radiate alternating magnetic fields into a region in proximity to the myocardium, and define a frame of reference 108 for the location and orientation determined by the sensor signals. The Carto® system referred to above uses such a magnetic tracking system.

Position sensors other than the coil type described above for sensor 46 are known in the art. For example, a position sensor may be configured from multiple electrodes, and currents traversing the electrodes, and/or voltages measured at the electrodes, may be used to determine the location and orientation of the position sensor. Furthermore, as described above, fiber optic 50 may be configured as a position sensor. For simplicity the following description assumes that sensor 46 is a coil type as described above, and that module 98 is configured to control magnetic field radiators 102, 104, and 106, and to receive and analyze the resulting signals from the sensor. The description may be adapted, *mutatis mutandis,* to include changing the functioning of distal end tracking module 98, if position sensor 46 is not a coil type, and/or if fiber optic 50 is configured as a position sensor.

Bank 90 also comprises an electrode tracking module 110 and an ultrasound module 112. Electrode tracking module 110 identifies and tracks the locations of electrodes 42 by any method known in the art, for example by measuring electrode voltages when the electrodes are in an electric field, and/or by measuring currents from the electrodes. Both of these systems use skin patches on patient 18, to generate the electric field, and to receive the currents from the electrodes. Electrodes 42 thus act as location sensors, and are herein also referred to as location sensors 42. The locations of location sensors 42 are measured relative to a frame of reference defined by the skin patches, and processor 66 registers this frame of reference with frame of reference 108 of radiators 102, 104, and 106, by methods well known in the art. For clarity the skin patches are not shown in Fig. 1.

Tracking module 110 is also configured to determine if a given identified electrode 42 contacts tissue 15, typically by observing a change in impedance between the identified electrode and a grounding patch (not shown in Fig. 1) when the electrode contacts the tissue. The Carto® system implements the electrode identification and tracking functionality, and the contact functionality, described herein.

Ultrasound module 112 comprises an ultrasound transducer 114 which generates ultrasound pulses, and the module conveys the pulses to fiber optic 50. Transducer 114 typically comprises a piezoelectric crystal. In one embodiment the pulses have an approximate length of 2 ns, and the frequency of a pulse is in a range between approximately 6 MHz and approximately 33 MHz. The pulses are emitted from fiber optic distal end 54, as described above. As is also described above, reflected pulses are received by distal end 54. These pulses are transferred to module 112 and to transducer 114, and the module is configured to measure the time of flight (TOF) of the pulses, between transmission from and reception by end 54.

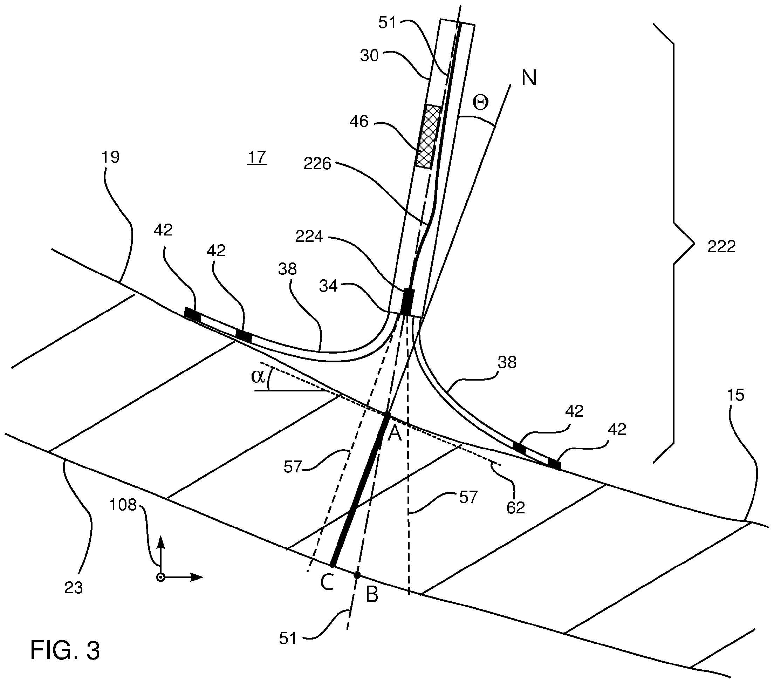

Fig. 3 is a schematic illustration of an alternate distal end 222 of probe 20, according to an embodiment of the present invention. Apart from the differences described below, the operation of distal end 222 is generally similar to that of distal end 22 (Fig. 2), and elements indicated by the same reference numerals in both distal end 22 and 222 are generally similar in construction and in operation.

In place of fiber optic 50, distal end 222 comprises an ultrasound transducer 224, which in some embodiments may comprise a piezoelectric crystal. Transducer 224 is substantially similar to transducer 114, and in embodiments comprising distal end 222, transducer 114 is not operative, and is typically not present, in module 112. Transducer 224 is located at termination 34 of element 30, and, as for distal end 54, transmits ultrasound pulses along axis 51. Transducer 224 is connected by cabling 226 to ultrasound module 112, and the module is configured to provide suitable signals via the cabling to cause transducer 224 to transmit the ultrasound pulses from end 34. The module is also configured to acquire, via the cabling, signals generated by transducer 224 in response to reflected ultrasound pulses received by the transducer. Thus, transducer 224 acts as an ultrasound transmission and receiving port, and is also referred to herein as transducer port 224.

As is also described above for distal end 22, for distal end 222 module 112 is configured to measure the TOF between transmission of the pulses and receipt of the reflected pulses via port 224.

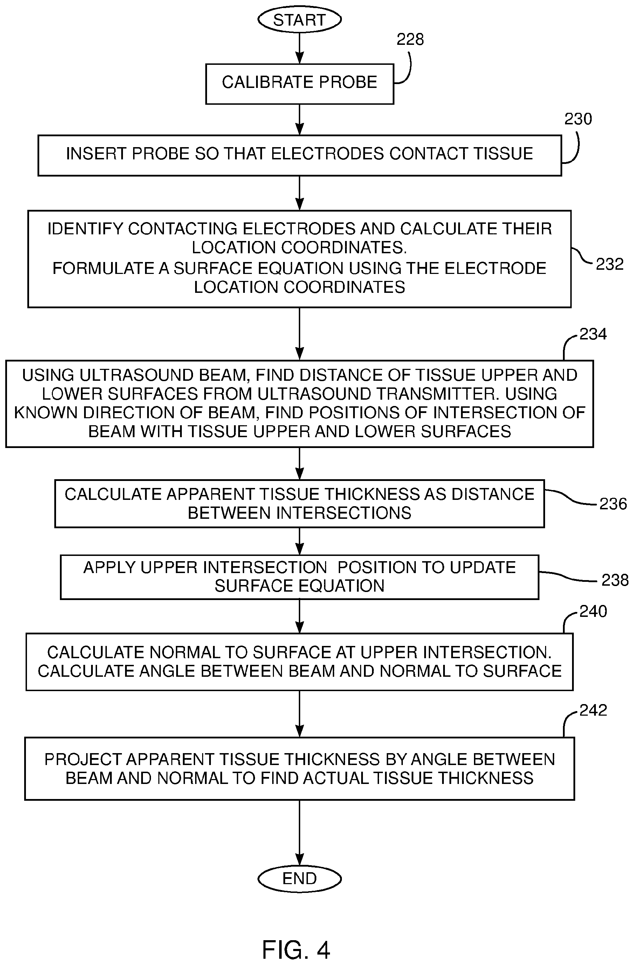

Fig. 4 is a flowchart of steps performed for operation of apparatus 12. Except as otherwise stated, in the following description of the steps probe 20 is assumed to have distal end 22, and the description may be adapted, *mutatis mutandis,* if probe 20 has distal end 222.

In a preparatory calibration step 228 probe 20 is calibrated, by methods well known in the art, so that processor 66 is able to equate signals from sensor 46 with the orientation of axis 51, and with the position of termination 34, measured in frame of reference 108.

In an initial procedure step 230 professional 14 inserts probe 20 into proximity with tissue 15, until a plurality, typically three or more, of electrodes 42 contact the tissue. Processor 66 and module 110 determine that electrodes 42 make contact with tissue 15, as described above, and the processor may present a notification of the contact to the professional on screen 82.

In a first surface definition step 232, processor 66 and module 110 identify each contacting electrode 42 and calculate their respective location coordinates in frame of reference 108. The processor then formulates an initial equation for a surface having the best fit to the location coordinates. In one embodiment the initial equation corresponds to that for a plane surface, as in equation (1) :

$$ax + by + cz = d \qquad (1)$$

where (x, y, z) are location coordinates of the electrodes, and a, b, c, and d are constants derived from substituting the coordinates into the equation.

In an ultrasound step 234, processor 66 invokes module 112 to generate an ultrasound pulse and emit it from transceiver port 54 at a time $T_0$, recorded by the processor. The pulse travels through the blood of region 17, along axis 51 so that axis 51 corresponds to the direction of the pulse. The pulse continues along axis 51 until it strikes a point A of proximal surface 19, so that point A is an intersection of the proximal surface with the axis. At point A, because of the change in characteristics between blood and tissue, causing a difference in the speed of sound in the two materials, the pulse splits so that there is a partially reflected pulse and a partially transmitted pulse.

A section of the reflected pulse is received at port 54, and the received pulse is detected by module 112, using transducer 114, which records a time $T_1$ at which the pulse is received. Using the time of flight of the pulse, $(T_1-T_0)$, and an assumed speed of sound in blood, the processor calculates a distance of point A from termination 34. In one embodiment the speed of sound in blood is assumed to be 1578 ms$^{-1}$.

In addition to finding the distance of A from termination 34, the processor also finds the direction of A from the termination, by finding the direction, i.e., the orientation, of axis 51 from signals acquired from sensor 46. As is explained above, the processor is able to calculate the position of termination 34 in frame of reference 108 from the sensor 46 signals. Thus, in step 234 processor 66 finds the position of point A in frame of reference 108.

As explained above, there is a partially transmitted pulse at point A, and this partially transmitted pulse continues along axis 51 until it strikes distal surface 23 at a point B. As for surface 19, because of the change in the speed of sound at surface 23, the pulse at B is partially reflected, and a portion of this reflected pulse travels back along axis 51 until it is received at port 54. The received pulse is detected by module 112, which records a time $T_2$ at which the pulse is received.

Using the overall time of flight of the pulse, $(T_2 - T_0)$, the calculated distance of A to termination 34, and an assumed speed of sound in tissue 15, the processor calculates a distance of point B from termination 34. In one embodiment the speed of sound in tissue is assumed to be 1540 ms$^{-1}$. Because point B is on axis 51, in step 234 the processor is also able to find the position of point B in frame of reference 108.

In an initial tissue thickness step 236, processor 66 calculates an apparent thickness of tissue 15 as the distance AB.

It will be understood that distance AB is typically not the thickness of the tissue, since usually line segment AB is not normal to surface 19. In the following steps the processor applies a correction to the value AB in order to find a corrected value of the tissue thickness.

In a second surface definition step 238, processor 66 applies the position of point A, determined in step 234, to update the equation for surface 19 determined in step 232. From the updated equation, processor 66 calculates an orientation of front surface 19 at point A, by determining an orientation angle $\alpha$ of a tangent surface 62 (shown as a broken line in the figure) to the front surface in frame of reference 108.

In a normal calculation step 240, from the updated surface equation and the orientation of the front surface found in step 238, the processor calculates a direction, measured in frame of reference 108, for a normal AN to surface 19 at point A. From the direction of normal AN, the processor calculates an inclination angle $\theta$ ($0 \le \theta \le 90°$) between the normal and axis 51. Typically $\theta < 45°$.

In a final adjust tissue thickness step 242, the processor projects the distance AB onto normal AN, so forming a line segment AC. Line segment AC has a corrected, actual, value for the tissue thickness, according to equation (2):

$$t = AB \cdot \cos\theta \qquad (2)$$

where t is the corrected tissue thickness, i.e., the length of segment AC,

AB is the apparent tissue thickness, and
$\theta$ is as defined above.

While the description above assumes that location sensors 42 comprise electrodes, it will be appreciated that the scope of the present invention comprises location sensors formed from other elements, such as coils generating location signals in response to magnetic fields from radiators 102, 104, and 106.

It will thus be appreciated that the embodiments described above are cited by way of example, and that the present invention is not limited to what has been particularly shown and described hereinabove. Rather, the scope of the present invention is defined by the claims .

## Claims

1. Apparatus for mapping endocardial sub-surface characteristics, the apparatus comprising:

   a probe (20) comprising a distal end (22, 222) configured to be brought into proximity with tissue of an internal organ of a human patient;
   a position sensor (46), attached to the distal end and configured to generate position signals indicative of a location and an orientation of the distal end;
   an ultrasonic transducer (114, 224) configured to transmit an ultrasound pulse in a direction of the orientation of the distal end and to receive reflections of the ultrasound pulse from the tissue;
   a plurality of location sensors (42), attached to the distal end of the probe and configured to contact a surface of the tissue (19) at respective locations in a vicinity of the distal end and to output respective location signals indicative of the respective locations; and
   a processor (66), configured to
   identify first and second reflections of the ultrasound pulse that were received respectively from front and rear surfaces of the tissue;
   process the location signals to find an orientation angle of the front surface of the tissue in the vicinity of the distal end; and
   estimate a thickness of the tissue along an axis normal to the front surface based on a time elapsed between receipt of the first and second reflections and an inclination of the direction of the orientation of the distal end relative to the orientation angle of the front surface.

2. The apparatus according to claim 1, and comprising a fiber optic (50) attached to the distal end, the fiber optic having a fiber optic proximal end, coupled to the ultrasonic transducer, and a fiber optic distal end (54), located at a distal termination (34) of the distal end, configured to transmit the ultrasound pulse and receive the reflections of the ultrasound pulse.

3. The apparatus according to claim 2, wherein the fiber optic comprises one or more optical gratings configured so that diffracted light from the gratings is indicative of the location and the orientation of the distal end, so that the one or more optical gratings act as a further position sensor.

4. The apparatus according to claim 1, wherein the position sensor comprises at least one coil configured to generate the position signals in response to a magnetic field traversing the sensor.

5. The apparatus according to claim 1, wherein the position sensor comprises one or more optical gratings formed in a fiber optic located in the distal end, and wherein diffracted light from the gratings is indicative of the location and the orientation of the distal end.

6. The apparatus according to claim 1, and comprising at least three flexible branches splaying from a termination of the probe distal end, and wherein the location sensors comprise electrodes attached to the flexible branches.

7. The apparatus according to claim 6, wherein at least one electrode (42) is attached to each flexible branch.

8. The apparatus according to claim 1, wherein the position sensor comprises at least one coil configured to generate the position signals in response to a magnetic field traversing the at least one coil.

9. The apparatus according to claim 1, and comprising at least three flexible branches splaying from a termination of the probe distal end, and wherein the location sensors comprise coils attached to the flexible branches.

10. The apparatus according to claim 9, wherein at least one coil is attached to each flexible branch.

11. The apparatus according to claim 1, wherein the processor is configured to calculate a position of an intersection of the front surface of the tissue with an axis of the probe based on a time of flight of the first reflection.

12. The apparatus according to claim 11, wherein the processor is configured to formulate an equation for the front surface based on the position of the intersection and the respective locations.

13. The apparatus according to claim 12, wherein the processor is configured to calculate the orientation angle of the front surface at the intersection using the equation.

14. The apparatus according to claim 1, wherein the processor is configured to estimate the thickness of the tissue as a projection of an apparent tissue thickness onto the axis, and wherein the processor calculates the apparent tissue thickness in response to the time elapsed.

15. The apparatus according to claim 1, wherein the internal organ comprises a heart of the human patient.


**Patentansprüche**

1. Gerät zum Mapping endokardialer Charakteristika unter der Oberfläche, wobei das Gerät umfasst:

   eine Sonde (20), umfassend ein distales Ende (22, 222), das konfiguriert ist, um in die Nähe von Gewebe eines inneren Organs eines menschlichen Patienten gebracht zu werden;
   einen Positionssensor (46), der an dem distalen Ende befestigt ist und konfiguriert ist, um Positionssignale zu generieren, die einen Ort und eine Orientierung des distalen Endes angeben;
   einen Ultraschallwandler (114, 224), der konfiguriert ist, um einen Ultraschallimpuls in eine Richtung der Orientierung des distalen Endes zu senden und Reflexionen des Ultraschallimpulses von dem Gewebe zu empfangen;
   eine Vielzahl von Ortssensoren (42), die an dem distalen Ende der Sonde befestigt und konfiguriert sind, um eine Oberfläche des Gewebes (19) an jeweiligen Orten in der Nähe des distalen Endes zu kontaktieren und jeweilige Ortssignale auszugeben, welche die jeweiligen Orte angeben; und
   einen Prozessor (66), der konfiguriert ist zum Identifizieren von ersten und zweiten Reflexionen des Ultraschallimpulses, die von vorderen beziehungsweise rückseitigen Oberflächen des Gewebes empfangen wurden;
   Verarbeiten der Ortssignale, um einen Orientierungswinkel der vorderen Oberfläche des Gewebes in der Nähe des distalen Endes zu finden; und
   Schätzen einer Dicke des Gewebes entlang einer Achse senkrecht zu der vorderen Oberfläche basierend auf einer Zeit, die zwischen dem Empfang der ersten und der zweiten Reflexionen verstrichen ist, und einer Neigung der Richtung der Orientierung des distalen Endes relativ zu dem Orientierungswinkel der vorderen Oberfläche.

2. Gerät nach Anspruch 1, und umfassend eine Faseroptik (50), die an dem distalen Ende befestigt ist, wobei die Faseroptik ein proximales Ende der Faseroptik, das an den Ultraschallwandler gekoppelt ist, und ein distales Ende der Faseroptik (54) aufweist, das sich an einem distalen Endteil (34) des distalen Endes befindet, und konfiguriert ist, um die Ultraschallimpulse zu senden und die Reflexionen der Ultraschallimpulse zu empfangen.

3. Gerät nach Anspruch 2, wobei die Faseroptik ein oder mehrere optische Gitter umfasst, die so konfiguriert sind, dass gebeugtes Licht von den Gittern den Ort und die Orientierung des distalen Endes angibt, so dass das eine oder die mehreren optischen Gitter als weiterer Positionssensor fungieren.

4. Gerät nach Anspruch 1, wobei der Positionssensor mindestens eine Spule umfasst, die konfiguriert ist, um die Positionssignale in Reaktion auf ein Magnetfeld zu generieren, das den Sensor quert.

5. Gerät nach Anspruch 1, wobei der Positionssensor ein oder mehrere optische Gitter umfasst, die in einer Faseroptik gebildet sind, die sich in dem distalen Ende befinden, und wobei gebeugtes Licht aus den Gittern den Ort und die Orientierung des distalen Endes angibt.

6. Gerät nach Anspruch 1, und umfassend mindestens drei flexible Zweige, die sich von einem Endteil des distalen Endes der Sonde aus spreizen, und wobei die Ortssensoren Elektroden umfassen, die an den flexiblen Zweigen befestigt sind.

7. Gerät nach Anspruch 6, wobei mindestens eine Elektrode (42) an jedem flexiblen Zweig befestigt ist.

8. Gerät nach Anspruch 1, wobei der Positionssensor mindestens eine Spule umfasst, die konfiguriert ist, um die Positionssignale in Reaktion auf ein Magnetfeld zu generieren, das die mindestens eine Spule quert.

9. Gerät nach Anspruch 1, und umfassend mindestens drei flexible Zweige, die sich von einem Endteil des distalen Endes der Sonde aus spreizen, und wobei die Ortssensoren Spulen umfassen, die an den flexiblen Zweigen befestigt sind.

10. Gerät nach Anspruch 9, wobei mindestens eine Spule an jedem flexiblen Zweig befestigt ist.

11. Gerät nach Anspruch 1, wobei der Prozessor konfiguriert ist, um basierend auf einer Lichtlaufzeit der ersten Reflexion eine Position eines Kreuzungspunkts der vorderen Oberfläche des Gewebes mit einer Achse der Sonde zu berechnen.

12. Gerät nach Anspruch 11, wobei der Prozessor konfiguriert ist, um eine Gleichung für die vordere Oberfläche basierend auf der Position des Kreuzungspunkts und der jeweiligen Orte zu formulieren.

13. Gerät nach Anspruch 12, wobei der Prozessor konfiguriert ist, um den Orientierungswinkel der vorderen Oberfläche an dem Kreuzungspunkt unter Verwendung der Gleichung zu berechnen.

14. Gerät nach Anspruch 1, wobei der Prozessor konfiguriert ist, um die Dicke des Gewebes als Projektion einer scheinbaren Gewebedicke auf die Achse zu schätzen, und wobei der Prozessor die scheinbare Gewebedicke in Reaktion auf die verstrichene Zeit berechnet.

15. Gerät nach Anspruch 1, wobei das innere Organ ein Herz des menschlichen Patienten umfasst.

## Revendications

1. Appareil pour cartographier des caractéristiques de subsurface de l'endocarde, l'appareil comprenant :

une sonde (20) comprenant une extrémité distale (22, 222) conçue pour être amenée à proximité du tissu d'un organe interne d'un patient humain ;
un capteur de position (46), fixé à l'extrémité distale et configuré pour générer des signaux de position faisant état d'un emplacement et d'une orientation de l'extrémité distale ;
un transducteur à ultrasons (114, 224) configuré pour transmettre une impulsion ultrasonore dans une direction de l'orientation de l'extrémité distale et pour recevoir des réflexions de l'impulsion ultrasonore en provenance du tissu ;
une pluralité de capteurs d'emplacement (42), fixés à l'extrémité distale de la sonde et configurés pour entrer en contact avec une surface du tissu (19) au niveau d'emplacements respectifs au voisinage de l'extrémité distale et pour émettre des signaux d'emplacement respectifs faisant état des emplacements respectifs ; et
un processeur (66), configuré pour

identifier des première et seconde réflexions de l'impulsion ultrasonore qui ont été reçues respectivement en provenance des surfaces avant et arrière du tissu ;

traiter les signaux d'emplacement pour trouver un angle d'orientation de la surface avant du tissu au voisinage de l'extrémité distale ; et

estimer une épaisseur du tissu le long d'un axe normal à la surface avant sur la base d'un temps écoulé entre la réception des première et seconde réflexions et une inclinaison de la direction de l'orientation de l'extrémité distale par rapport à l'angle d'orientation de la surface avant.

2. Appareil selon la revendication 1, et comprenant une fibre optique (50) fixée à l'extrémité distale, la fibre optique ayant une extrémité proximale de fibre optique, couplée au transducteur à ultrasons, et une extrémité distale (54) de fibre optique, située au niveau d'une terminaison distale (34) de l'extrémité distale, configurée pour transmettre l'impulsion ultrasonore et recevoir les réflexions de l'impulsion ultrasonore.

3. Appareil selon la revendication 2, dans lequel la fibre optique comprend un ou plusieurs réseaux optiques configurés de sorte que la lumière diffractée à partir des réseaux soit révélatrice de l'emplacement et de l'orientation de l'extrémité distale, de sorte que le ou les réseaux optiques agissent comme un capteur de position supplémentaire.

4. Appareil selon la revendication 1, dans lequel le capteur de position comprend au moins une bobine configurée pour générer les signaux de position en réponse à un champ magnétique traversant le capteur.

5. Appareil selon la revendication 1, dans lequel le capteur de position comprend un ou plusieurs réseaux optiques formés dans une fibre optique située dans l'extrémité distale, et dans lequel la lumière diffractée depuis les réseaux fait état de l'emplacement et de l'orientation de l'extrémité distale.

6. Appareil selon la revendication 1, et comprenant au moins trois branches flexibles s'évasant depuis une terminaison de l'extrémité distale de sonde, et dans lequel les capteurs d'emplacement comprennent des électrodes fixées aux branches flexibles.

7. Appareil selon la revendication 6, dans lequel au moins une électrode (42) est fixée à chaque branche flexible.

8. Appareil selon la revendication 1, dans lequel le capteur de position comprend au moins une bobine configurée pour générer les signaux de position en réponse à un champ magnétique traversant l'au moins une bobine.

9. Appareil selon la revendication 1, et comprenant au moins trois branches flexibles s'évasant depuis une terminaison de l'extrémité distale de sonde, et dans lequel les capteurs d'emplacement comprennent des bobines fixées aux branches flexibles.

10. Appareil selon la revendication 9, dans lequel au moins une bobine est fixée à chaque branche flexible.

11. Appareil selon la revendication 1, dans lequel le processeur est configuré pour calculer une position d'une intersection de la surface avant du tissu avec un axe de la sonde sur la base d'un temps de vol de la première réflexion.

12. Appareil selon la revendication 11, dans lequel le processeur est configuré pour formuler une équation pour la surface avant sur la base de la position de l'intersection et des emplacements respectifs.

13. Appareil selon la revendication 12, dans lequel le processeur est configuré pour calculer l'angle d'orientation de la surface avant au niveau de l'intersection en utilisant l'équation.

14. Appareil selon la revendication 1, dans lequel le processeur est configuré pour estimer l'épaisseur du tissu en tant que saillie d'une épaisseur apparente du tissu sur l'axe, et dans lequel le processeur calcule l'épaisseur apparente du tissu en réponse au temps écoulé.

15. Appareil selon la revendication 1, dans lequel l'organe interne comprend le coeur du patient humain.

FIG. 1

FIG. 2

EP 3 662 841 B1

FIG. 3

START

CALIBRATE PROBE — 228

INSERT PROBE SO THAT ELECTRODES CONTACT TISSUE — 230

IDENTIFY CONTACTING ELECTRODES AND CALCULATE THEIR LOCATION COORDINATES.
FORMULATE A SURFACE EQUATION USING THE ELECTRODE LOCATION COORDINATES — 232

USING ULTRASOUND BEAM, FIND DISTANCE OF TISSUE UPPER AND LOWER SURFACES FROM ULTRASOUND TRANSMITTER. USING KNOWN DIRECTION OF BEAM, FIND POSITIONS OF INTERSECTION OF BEAM WITH TISSUE UPPER AND LOWER SURFACES — 234

CALCULATE APPARENT TISSUE THICKNESS AS DISTANCE BETWEEN INTERSECTIONS — 236

APPLY UPPER INTERSECTION POSITION TO UPDATE SURFACE EQUATION — 238

CALCULATE NORMAL TO SURFACE AT UPPER INTERSECTION. CALCULATE ANGLE BETWEEN BEAM AND NORMAL TO SURFACE — 240

PROJECT APPARENT TISSUE THICKNESS BY ANGLE BETWEEN BEAM AND NORMAL TO FIND ACTUAL TISSUE THICKNESS — 242

END

FIG. 4

**EP 3 662 841 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1720038 A2
- WO 2014064577 A1
- US 2003013958 A1
- US 2011028848 A1
- WO 2012122517 A2

**Non-patent literature cited in the description**

- **CULSHAW B et al.** The detection of ultrasound using Fiber-Optic Sensors. *IEEE sensors journal,* 01 July 2008, vol. 8 (7), 1360-1367